# EUROPEAN PATENT APPLICATION

(11) **EP 1 270 740 A1**
(43) Date of publication of application: **02.01.2003**
(21) Application number: 01115946.4
(22) Date of filing: 29.06.2001
(51) Int. Cl.: C12Q 1/68

(54) **Biochip and its use for determining inflammation**

(71) Applicant: SIRS-Lab GmbH, 07745 Jena (DE)
(72) Inventor: Zipfel, Peter Franz, 22547 Hamburg (DE); Saluz, Hans-Peter, 07646 Oberbodnitz (DE); Russwurm, Stefan, 07743 Jena (DE); Reinhart, Konrad, 07743 Jena (DE)
(74) Representative: Störle, Christian, Dr.

(57) **Abstract**

The application relates to a nucleic acid and/or protein chip comprising probe nucleic acids and/or probe proteins, which are specific for cellular stress, inflammatory and immune reactions, associated with stress, inflammatory and immune reactions, induced during acute phase responses or any combination thereof and which are immobilised on a carrier in an orderly grid pattern. Furthermore, the use of such a chip for the diagnosis of stress responses or inflammation, for monitoring the course of inflammation or for detecting the severity of inflammation is described.

## Description

The invention relates to a nucleic acid and/or protein chip and its use for the diagnosis of inflammation, for monitoring the course of inflammation, for detecting the severity of inflammation and determining the individual prognosis.

Inflammation occurs frequently as a symptom of various diseases, for instance cancer or infection by micro-organisms. Thus, the accurate and fast diagnosis as well as the therapy of inflammation are some of the major problems in the field of medicine.

Sepsis, which also occurs frequently, can be caused by various microbes but also other reasons and it results in inflammatory reactions.

For the therapy of inflammation and/or the disease which causes the inflammation, it is necessary to diagnose the inflammation carefully and to obtain information regarding the cause of the inflammation, for example of the infectious agent.

It is in particular necessary that the diagnosis of inflammation can be carried out very quickly in order to start with the appropriate therapy soon and, furthermore, in order to analyse a huge number of samples from patients in a very short time.

Thus, the technical problem underlying the present invention is to provide means for the rapid determination of inflammation and related responses of patients.

This technical problem is solved by a nucleic acid and/or protein chip (biochip) comprising probe nucleic acids and/or probe proteins, which are specific for cellular stress, inflammatory and immune reactions, associated with stress, inflammatory and immune reactions, induced during acute phase responses or any combination thereof and which are immobilised on a carrier in an orderly grid pattern.

The term "probe nucleic acids and/or probe proteins" as used according to the present invention refers to nucleic acids only, proteins only and also to the simultaneous presence of nucleic acids and proteins.

According to the present invention, the term "nucleic acids" generally designates DNA and RNA of any kind and from any source. As DNA molecules cDNA can be used which can be generated from cellular or isolated mRNA.

The present invention relates to the nucleic acid chip technology, which is a very new research area, wherein the nucleic acid chips having up to several thousand spots of nucleic acid can be used. To this end, nucleic acids are immobilised on a carrier in an orderly grid pattern. The DNA or RNA to be examined (target nucleic acids or sample) is generally labelled, e.g. by using a fluorescent dye, and applied to the chip. In the case of hybridization of target nucleic acids to the probe nucleic acids bound to the carrier and having complementary sequences with the DNA or RNA to be examined a signal is detected at a corresponding position within that grid pattern, for example through a CCD camera or by a laser scanner, in a usual way.

In other words, the afore-described chip technology is based on the knowledge that the expression of a panel of molecules can be analysed simultaneously by hybridizing RNA or DNA molecules to probe nucleic acids immobilised on a carrier in an orderly grid pattern.

The present invention is described hereinafter in detail by referring to nucleic acid chips, but it should be understood that it is not limited thereto. The present invention can also be applied to protein chips where probe proteins are immobilised in an orderly grid pattern on a carrier. Proteins in the sample to be investigated can be detected by interaction with the probe proteins. This can be carried out in a usual way. The skilled artisan also knows chemicals, materials and methods to prepare protein chips and to use it accordingly, or chemicals, materials and methods suitable for protein chips can be developed in view of the details given hereinafter in connection with nucleic acid chips.

In a preferred embodiment of the present invention, the probe nucleic acids comprise at least one compound selected from a gene or a gene-product of immune mediators, transcription factors, acute phase proteins, complement components, molecules of the coagulation system, adhesion molecules, markers for cell specifity, housekeeping genes, molecules associated with body response to infection and sepsis, genes derived from infectious agents, like microbes, splice variants of said genes and fragments of said genes.

The term "fragment" as used herein refers to nucleic acid sequences which are shorter than the gene but still have the characteristics of said gene leading to a protein having basically the same chemical and/or physical properties as the wild type protein.

Examples of the immune mediators are growth factors, such as PDGF-α, -β, insuline like growth factor (ILGF), pro- and anti-inflammatory cytokines, such as interleukines, like IL-1 to IL-18, chemokines like platelet factor- 4 (PF-4), γ-interferon induced protein 10 (IP 10), growth related proteins Gro-α,-β and -γ, eotaxin, Mip-1α, Mip-1β, RANTES, growth factor receptors and other cell growth-related antigens.

The transcription factors can be EGR-, NFAT- and NFkB-proteins, and members of the AP-1 and CREB protein family.

Other acute phase proteins include C-reactive proteins and tissue factor 1.

Examples of the complement components are factor H, FHL-1 and FHR encoding genes.

Markers for cell specifity include T cell receptor genes and genes associated with the T cell receptor, such as CD3, CD14, CD11e/CD18, CD28, CD143 proteins, genes coding for cell specific markers such as CD4 and CD8 for T cell subtypes, CD14 for macrophages, IgM for B cells.

Representatives of housekeeping genes are glucose-6-phosphate-dehydrogenase, actin and glycerinaldehyde-3-phosphate-dehydrogenase.

The additional molecules associated with body response to infection and sepsis include as examples the various transcripts of the human *Calc* genes, representing transcripts coding for procalcitonin, catalacin N-terminal peptide, catacalin as well as the calcitonin gene related peptide and products thereof.

Inflammation is very often caused by infectious agents like microbes. For a suitable therapy directed against the cause of the inflammation, e. g. the microbes, it is desirable to obtain as much information as possible regarding the microbes. Thus, proteins or genes of the microbes can be fixed on the carrier of the biochip according to the present invention to obtain such information.

The biochip according to the present invention can contain one of the afore mentioned probes or any combination up to all mentioned probes. The selection of the probes is dependent on the kind of inflammation to be investigated and it can be easily determined by a person skilled in the art depending on the kind of disease to be evaluated.

The nucleic acid chip and the protein chip according to the present invention have a plurality of advantages. One advantage is that, in a very easy and fast way, the determination of inflammation of any kind can be carried out, in particular by using the explicitly afore-mentioned probe nucleic acids. The determination can be done in an accurate manner. Furthermore, the infectious agent and/or the reactions of the body caused by an infectious agent can be measured directly by detecting genes, gene fragments and/or proteins. By using the biochips according to the present invention, the diagnosis of inflammation can be standardised. The pattern of expressed or defined genes is useful not only for the diagnosis of inflammation, but also for the early detection of it, for monitoring it during therapy and for the detection of the severity of it. In addition, the biochip according to the present invention allows a survey of the progression of the disease, the progress of the inflammation and the success of its treatment.

For using the biochip according to the present invention, DNA or RNA to be examined can be labelled, for example by a fluorescent dye, and applied to the chip. The hybridization of the DNA or RNA to be examined to the probe molecules bound to the carrier having complementary sequences can be detected at the corresponding position within the grid through usual means, for example a CCD camera or by a laser scanner.

The biochip according to the present invention allows an automative analysis and, furthermore, the amount of samples to be investigated can be determined quantitatively, which makes it possible to give more accurate information on the course of an inflammation and in particular on the success of its treatment.

As already mentioned above, the probe nucleic acids and/or the probe proteins having the afore-mentioned specifities are immobilised on the carrier. This means that either nucleic acids only, proteins only or nucleic acids together with proteins are bound to the carrier.

According to the biochip of the present invention, the probes are immobilised on the carrier in an orderly grid pattern as usually carried out in the chip technology as described above. An orderly grid pattern includes areas on the carrier containing the probes for example as a spot. Nucleic acid chips and protein chips are generally provided with a plurality of areas on the carrier. In this case, all areas can have the same probe or the areas can have different probes, i. e. nucleic acid probes and/or protein probes.

In a preferred embodiment of the biochip according to the present invention, the probe nucleic acids and/or probe proteins are immobilised on the carrier in predetermined areas. This means that the area on which the probes shall be immobilised, is known in advance. It also means that the location on which a specific probe is immobilised on the carrier is known before the chip is used, for example treated with the samples to be investigated. This advantageously allows a standardisation and automation of the determination of inflammation. Furthermore, the determination is carried out in a very easy and fast way.

In a further preferred embodiment of the biochip according to the present invention, the areas are spaced from each other. This provides, in a very advantageous manner, better and more accurate results and, furthermore, an automation and standardisation of the determination of inflammation is possible.

Preferably, the orderly grid pattern of the probes is arranged in the form of parallel rows of the areas. Examples of such a carrier are microtiter plates, where the wells represent the areas for the probes, and which are generally arranged in parallel rows. According to this embodiment, an automation and standardisation of the determination of inflammation is achieved in an advantageous manner.

In a preferred embodiment, the carrier is a glass slide. It has favourable optical characteristics and, furthermore, it has the advantages of being solid, and not fluorescent so that it is in particular useful for the fast and accurate determination of inflammation due to these properties.

One method for the preparation of the biochip according to the present invention shall be described hereinafter.

For the fabrication of the biochip, probes, in particular nucleic acids, can be solved in spotting solutions and thereafter they can be spotted on the carrier by a usual spotting process. As a spotting solution, saline sodium citrate (SSC) buffer is widely used. It can be suplemented with 50 % dimethyl sulfoxide.

The binding of the probes, e. g. of the nucleic acids and/or the proteins, to the carrier can be achieved, either by covalent bounds or electrostatical (ionical) bounds.

For covalent binding of nucleic acids, for example compounds providing aldehyde groups are coated on the surface of the carrier. Nucleic acids contain primary amino groups which can react with the aldehyde group to form a Schiff base, i.e. a covalent bond.

For the electrostatical binding in particular of nucleic acids, use is made of the fact that they are generally negatively charged. By providing positive charges on the surface of the carrier, binding between the negatively charged nucleic acids and the positively charged surface of the carrier can be achieved by an interaction of the charges. For this purpose, glass surfaces coated with compounds providing positive charges, e. g. coated with poly-L-lysine and/or aminosilane are used. Such activated slides are well known in the art.

If necessary, cross linking of the nucleic acids with the carrier can be achieved by UV radiation.

As mentioned above, the biochip according to the present invention is useful for any kind of determination of inflammation or cellular stress.

Therefore, the biochip as mentioned above can be used for the diagnosis of cellular stress, of inflammation, for monitoring the course of inflammation, for detecting the severity of inflammation or determining the individual prognosis.

For this use, labelled mRNA, cDNA or proteins derived from humans or animals to be examined can be hybridised to the probe nucleic acids and/or probe proteins of the biochip and the hybridization pattern can be determined as already described above. Samples to be investigated can be isolated from peripheral blood cells from blood samples, in particular from venous blood, tissue samples, organs or organisms. The samples can be either used directly or can be further purified to obtain subgroups of hematologic cell population. The blood cells can be lysed and the RNA can be isolated in the usual way. The mRNA may be further purified and cDNA can be synthesized from mRNA according to standard procedures.

Advantageously, the sample cDNA is derived from fractionated cells or from purified mRNA. Furthermore, the sample nucleic acid can be derived from PCR generated fragments. These samples show the advantage that particularly strong signals are provided.

The cDNA or mRNA used as samples can be labelled with a marker. Any substance can be used as marker which can be detected after hybridization of the samples to the probes of the biochip. Examples of such markers are fluorescent dyes. The labelled cDNA or labelled mRNA can be hybridised to the probes of the biochip in the usual way. Protein analysis can be performed by standard protein arrays technologies accordingly.

Hereinafter, the use of the biochip according to the present invention is described in more detail with reference to nucleic acids but it should be understood that the present invention is not limited to nucleic acids but can also be applied to proteins.

Cells from humans or animals to be investigated regarding inflammation can be isolated from blood samples and peripheral blood mononuclear cells by standard procedures such as Ficoll Hypaque centrifugation or by Lymphoprep. In addition, tissues, organs or organisms such as infectious agents isolated from the patient plasma and from other biological sources can be used. The isolated cells can be used to enrich specific cell subtypes such as monocytes, T cell subtypes or B cell subtypes.

In order to purify the cells, they can be further enriched by standard protocols such as FACS sorting, magnet sorting or by lysing specific subtypes of cells. From the cells, tissues, or organisms, total RNA or fractions thereof can be isolated also by standard procedures, such as extraction by means of phenol/chloroform, RNAzol or Trizol.

The isolated RNA may be further purified to mRNA to enrich the number of coding molecules. This can be performed, for example, by oligo-dT selection by means of magnetic beads or by other procedures.

cDNA synthesis can also be achieved according to standard procedures. The converted cDNA may be further enriched by temperature cycling of the biological material by polymerase chain reaction using gene specific primers in combination with or in absence of oligo-dT primers.

For preparing a DNA array a carrier such as a Borofloat-33 glass slide can be coated by a thin layer of polymer useful for immobilising the DNA, such as a silane, containing reactive groups for the immobilisation of the DNA.

The hybridization of the labelled complementary RNA or a labelled complementary cDNA to the nucleic acids immobilised on the carrier can be performed according to standard protocols.

The labelling of the nucleic acids and the proteins contained in the sample to be invetigated can be performed in the usual way such as incorporation reactions, for example incorporation of Cy3- or Cy5-labelled Deoxynucleotides into cDNA by means of RTPCR or related reactions.

The signal detection can be performed by scanning for example with a ScanArray 2000.

The data and the quantitative expression pattern of the gene or the collection of genes can be further analysed and evaluated, for example, electronically by biocomputing using the appropriate software. For this, a computer program can be used to define a correlation of the expression of magnitude of expression of a specific gene or a group of genes with the severity of the inflammatory reactions as determined by assessing laboratory parameters or hemodynamic parameters. Such a program can be used to determine and predict the course of the severity or progression of the disease. In addition, it allows to follow the progress and effects of a therapeutic approach and intervention.

Protein arrays can be performed accordingly. However, for catching and visualisation, protein/protein-interactions can be used.

According to a preferred embodiment of the use of the present invention, the concentration of the hybridised labelled mRNA, cDNA or protein is determined. This means, in other words, that the level of nucleic acids or proteins in the sample to be investigated is determined quantitatively, which in turn allows easily to follow the course of an inflammation, for example to see whether or not the treatment of the inflammation is successful. If it is successful, the level of expressed nucleic acids or proteins in the sample decreases, and accordingly the detected signal is lowered.

The detection of the hybridised nucleic acid or proteins can be carried out, for example, by laser scanning or CCD equipment, and multi factorial bio information analysis can be included to define an association of certain genes or the expression level of certain genes or proteins with the severity of that disease. Severity of acute and inflammatory reactions such as sepsis can be defined, for example, by clinical evaluation such as APACHE II scoring.

In a preferred embodiment, the biochip according to the present invention can be used in relation to acute or chronic inflammation and, more preferably, for determining inflammation caused by sepsis or sepsis related syndromes. For detecting these diseases, the biochip according to the present invention is in particular useful and allows a fast and accurate determination.

Particularly, the severity of the inflammatory response in a particular set of patients can be determined by assessing well known laboratory and hemodynamic parameters to assess the course, the severity or progression of the disease. The analysis can be performed, for example, by relevant score systems such as APACHE II score.

The invention will be described hereinafter in more detail with reference to the example but it is to be understood that the example is intended only to illustrate the invention but not to limit it thereto.

### Example 1: Preparation of a biochip according to the present invention

Whole blood is collected by venal puncture from sepsis patients which are grouped according to the APACHE II score. In addition blood is collected from healthy human volunteers. The samples are collected in commercially available vacutainers, which contain a solution used to stabilize RNA according to standard procedures. RNA is isolated according to the protocoll of the supplier and mRNA is isolated according to standard protocolls (http://www.Microarrays.org/pdfs/YeastPolyAlsolation.pdf). The cDNA is labeled by incorporating Cy3 and Cy5 labeled nucleotides by using RT-transcriptase (http://www.Microarrays.org/pdfs/HumanRNALabel.pdf). The entire human cDNA library containing ca. 75 000 clones was obtained from RZPD (Heidelberg) and spotted according to standard procedures to poly-L-lysine coated glass slides. For spotting 200 micronpins (200 µm) (Telechem) an gene array was used according to standard procedures. The above described labeled molecules to be investigated are used for hybridization to the cDNA clones immobilized on the activated carrier surface as described (http://www.Microarrays.org/pdfs/Hybridization.pdf). Washing was exactly followed according to the protocol (http://www.Microarrays.org/pdfs/ArrayWashing.pdf). A Scan Array 3000 machine was used to detect the fluorecently labeled hybridized molecules according to the instruction manual. Data were compared between material obtained with Sepsis Patient and healthy human volunteers and AIDA ray test software was used for data analysis to determine up- or down regulated genes. Such genes are studied for expression in PBMC by standard Northern blot asays to further confirm their specific expression in the disease process (Sambrook et al. Molecular Cloning).

## Claims

1. Nucleic acid and/or protein chip comprising probe nucleic acids and/or probe proteins, which are specific for cellular stress, inflammatory and immune reactions, associated with stress, inflammatory and immune reactions, induced during acute phase responses or any combination thereof and which are immobilised on a carrier in an orderly grid pattern.

2. Chip according to claim 1, wherein the probe nucleic acid comprises at least one compound selected from a gene or a gene product selected from immune mediators, transcription factors, acute phase proteins, complement components, molecules of the coagulation system, adhesion molecules, markers for cell specifity, housekeeping genes, molecules associated with body response to infection and sepsis, genes derived from infectious agents, splice variants of said genes and fragments of said genes.

3. Chip according to claim 1 or 2, wherein the probe nucleic acids and/or probe proteins are immobilised on the carrier in predetermined areas.

4. Chip according to claim 3, wherein the areas are spaced from each other.

5. Chip according to any of the preceding claims, wherein the orderly grid pattern has the form of parallel rows of the areas.

6. Chip according to any of the preceding claims, wherein the carrier is a glass slide, a microtiter or nanotiter plate.

7. Use of a chip according to any of claims 1 to 6 for the diagnosis of cellular stress, of inflammation, for monitoring the course of inflammation, for detecting the severity of inflammation or determining the individual prognosis.

8. Use according to claim 7, wherein labelled mRNA, labelled cDNA or labelled proteins derived from humans or animals to be investigated are hybridised to the probe nucleic acid and/or probe proteins of the chip and the hybridization pattern is determined.

9. Use according to claim 8, wherein the concentration of the hybridised labelled mRNA, cDNA or protein is determined.

10. Use according to claim 7 or 8, wherein the inflammation is an acute or chronic inflammation.

11. Use according to any of claims 7 to 9, wherein the inflammation is caused by sepsis or sepsis related syndrome.
